(19) Europäisches Patentamt
European Patent Office
Office européen des brevets

(11) **EP 3 057 505 B1**

(12) **EUROPEAN PATENT SPECIFICATION**

(45) Date of publication and mention
of the grant of the patent:
**13.12.2017 Bulletin 2017/50**

(51) Int Cl.:
*A61B 5/11* (2006.01)    *A61B 5/22* (2006.01)
*A61B 5/00* (2006.01)    *G06F 19/00* (2011.01)
*G06K 9/00* (2006.01)    *G09B 19/00* (2006.01)
*G01P 7/00* (2006.01)

(21) Application number: **13815848.0**

(22) Date of filing: **18.10.2013**

(86) International application number:
**PCT/IT2013/000291**

(87) International publication number:
**WO 2015/056280 (23.04.2015 Gazette 2015/16)**

(54) **METHOD AND APPARATUS FOR ASSESSING THE PERFORMANCES OF AN ATHLETE THAT PERFORMS A GYMNASTIC EXERCISE**

VERFAHREN UND VORRICHTUNG ZUR BEURTEILUNG DER LEISTUNGEN EINES SPORTLERS BEI EINER TURNÜBUNG

PROCÉDÉ ET APPAREIL POUR ÉVALUER LES PERFORMANCES D'UN ATHLÈTE RÉALISANT UN EXERCICE DE GYMNASTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.08.2016 Bulletin 2016/34**

(73) Proprietor: **Beast Technologies S.R.L.**
**25122 Brescia (IT)**

(72) Inventors:
• **FINADRI, Tommaso**
**I-46043 Castiglione delle Stiviere (MN) (IT)**
• **PINZONI, Lucio**
**I-25015 Desenzano del Garda (BS) (IT)**
• **HAENDLER, Ernst-Vittorio**
**20143 Milan (IT)**

(74) Representative: **Ciceri, Fabio et al**
**Perani & Partners S.p.A.**
**Piazza San Babila, 5**
**IT-20122 Milano (IT)**

(56) References cited:

• **TAKENOSHITA K ET AL: "Development of a Portable Acceleration Monitor Device and its clinical application for the Quantitative Gait Assessment of the Elderly", ENGINEERING IN MEDICINE AND BIOLOGY SOCIETY, 2005. IEEE-EMBS 2005. 27T H ANNUAL INTERNATIONAL CONFERENCE OF THE SHANGHAI, CHINA 01-04 SEPT. 2005, PISCATAWAY, NJ, USA,IEEE, 1 September 2005 (2005-09-01), pages 3534-3537, XP010908573, DOI: 10.1109/IEMBS.2005.1617242 ISBN: 978-0-7803-8741-6**
• **XIAOPING YUN ET AL: "Self-contained Position Tracking of Human Movement Using Small Inertial/Magnetic Sensor Modules", 2007 IEEE INTERNATIONAL CONFERENCE ON ROBOTICS AND AUTOMATION - 10-14 APRIL 2007 - ROMA, ITALY, IEEE, PISCATAWAY, NJ, USA, 10 April 2007 (2007-04-10), pages 2526-2533, XP031389170, ISBN: 978-1-4244-0601-2**
• **A.M. SABATINI ET AL: "Assessment of Walking Features From Foot Inertial Sensing", IEEE TRANSACTIONS ON BIOMEDICAL ENGINEERING, vol. 52, no. 3, 1 March 2005 (2005-03-01), pages 486-494, XP055009157, ISSN: 0018-9294, DOI: 10.1109/TBME.2004.840727**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 057 505 B1

- LUINGE H J ET AL: "MEASURING ORIENTATION OF HUMAN BODY SEGMENTS USING MINIATURE GYROSCOPES AND ACCELEROMETERS", MEDICAL AND BIOLOGICAL ENGINEERING AND COMPUTING, SPRINGER, HEILDELBERG, DE, vol. 43, no. 2, 1 March 2005 (2005-03-01), pages 273-282, XP001508001, ISSN: 0140-0118, DOI: 10.1007/BF02345966

**Description**

*Field of the invention*

The present disclosure relates to a method and an apparatus for assessing the performances of an athlete that performs a gymnastic exercise, as defined in the preambles of claims 1 and 7 respectively.

Particularly, the present disclosure relates to a method and an apparatus for assessing the performances of an athlete and informing the athlete thereof during the gymnastic exercise.

*Discussion of Related Art*

In the prior art, methods and devices are known to be used to optimize the performance of a gymnastic exercise (or training session) of an athlete according to his/her muscular characteristics.

For example, EP 1834583 discloses a method and a device for evaluating the muscular capacity of athletes using short tests, such as squats and jumps.

Particularly, the object of the above disclosed invention is to measure kinematic parameters in a short-time effort and to display one or more quantities indicative of the muscular capacity of the athlete at the end of the test, i.e. without waiting too much time from the end of the gymnastic excercise.

For this purpose, EP 1834583 discloses the use of a three-axis accelerometer allowing acceleration measurement in the direction of weight transfer, i.e. a vertical direction.

Nevertheless, this method requires calibration prior to every exercise to determine the vertical axis along which the weight will be transferred.

Furthermore, before each exercise, the athlete is required to be still, e.g. for two seconds, and wait until he/she receives a confirmation tone by the sensor before starting the exercise.

Finally, it shall be noted that the method as disclosed in EP 1834583 at most provides indications about the muscular capacity of the athlete at the end of the exercise (or "short test") and not during performance of the exercise. Therefore, the athlete is only informed at the end of the gymnastic exercise, and there is no way of acting upon and/or assessing his/her performances during the exercise.

The article "Development of a Portable Acceleration Monitor Device and its clinical application for the Quantitative Gait Assessment of the Elderly" by K. Takenoshita et al., Proceedings of the 2005 IEEE Engineering in Medicine and Biology 27th Annual Conference, pp. 3534-3537, which is regarded as the closest prior art to the present invention, discloses a portable monitor for gait assessment using integration and regression of accelerometer data, and subtraction of the regression curve data.

*SUMMARY OF THE INVENTION*

The object of the invention is to provide a method and an apparatus that can obviate the drawbacks of the prior art, as defined in claims 1 and 7, respectively. One embodiment provides a method and an apparatus that can provide indications about the muscular capacity of an athlete during performance of an exercise and not only at the end of it.

Furthermore, the present invention can provide a method and a device that impose no limit on the duration or type of tests, whereby tests may be also other than raises and jumps, and last a longer time.

Also, the method and apparatus of the invention determine both acceleration and attitude, using a gyroscope and a magnetometer; thus, the method and apparatus of the invention afford non-intrusive acquisition of training data, without requiring calibration before each exercise.

Furthermore, with the present invention, while the inventive method and apparatus require no calibration, they afford higher accuracy, as they can correct any measurement error.

Finally, the method and apparatus of the present invention afford the following advantages:

- determining the maximum for each athlete without subjecting him/her to excessive loads, i.e. using a lower weight than the maximum load, as the athlete may simply perform a repetition to his/her maximum capacity using a weight he/she is used to, the maximum load being calculated by the apparatus with the inventive method;
- suggesting the right weight to be used by the athlete;
- checking that the movements performed during training are useful to achieve the desired goal.

*BRIEF DESCRIPTION OF THE DRAWINGS*

The characteristics and advantages of the present disclosure will appear from the following detailed description of possible practical embodiments thereof, which are shown as non-limiting examples in the drawings, in which:

- Figure 1 is a principle view of the apparatus of the present invention;
- Figure 2 shows a graphical icon representative of a parameter assessed by the apparatus and method of the present invention;
- Figure 3 shows a representation of the coordinate axes of the measuring device of the present invention;
- Figure 4 shows a flow diagram of a first embodiment of the method of the present invention;
- Figure 5 shows a chart of the operation of a few steps of the method as shown in the diagram of Figure 4, according to the present invention;
- Figure 6 shows a chart of the operation of further steps of the method as shown in the diagram of Figure 4, according to the present invention;

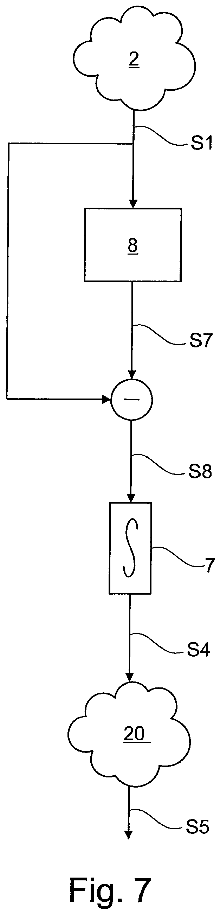

- Figure 7 shows a flow diagram of a second embodiment of the method of the present invention.

*DETAILED DESCRIPTION*

Although this is not expressly shown, the individual features described with reference to each embodiment shall be intended as auxiliary and/or interchangeable with other features, as described with reference to other embodiments.

Referring to the accompanying figures, numeral 1 designates the apparatus for assessing the performances of an athlete that performs a floor exercise or uses equipment for performing a gymnastic exercise.

The term equipment for performing a gymnastic exercise is intended to designate equipment such as barbells, squat machines or the like, that allow performance and/or repetition of a gymnastic exercise.

The term gymnastic exercise is intended to designate a set of activities performed to exercise, develop and strengthen a given ability of the athlete's body.

The apparatus 1 comprises a measuring device 2, having fastener means 3 for removably fastening the measuring device 2 to the body of the athlete (e.g. wrist, ankle or torso) or to the equipment designed for performance of such gymnastic exercise.

The apparatus 1 comprises a display 4 and processing means 5 in signal communication with the self-powered measuring device 2 and with the display.

The fastener means 3 preferably comprise a magnetic coupling and, in addition to or instead of it, a strap, Velcro, or other fastening devices known to the skilled persons, and adapted for removable connection of the self-powered measuring device 2 to the body of the athlete or the equipment for performing the gymnastic exercise.

The measuring device 2 is preferably self-, i.e. battery-powered. Preferably, the battery is a rechargeable lithium battery.

Advantageously, the measuring device 2 comprises a radio-frequency transceiver module 6 for establishing signal communication between the self-powered measuring device 2 and the display 4, although such signal communication may be supposed to be established also or only using data connection cables.

Preferably, the radio-frequency transceiver module is a wireless communication module that uses the Bluetooth protocol or a Wi-Fi standard.

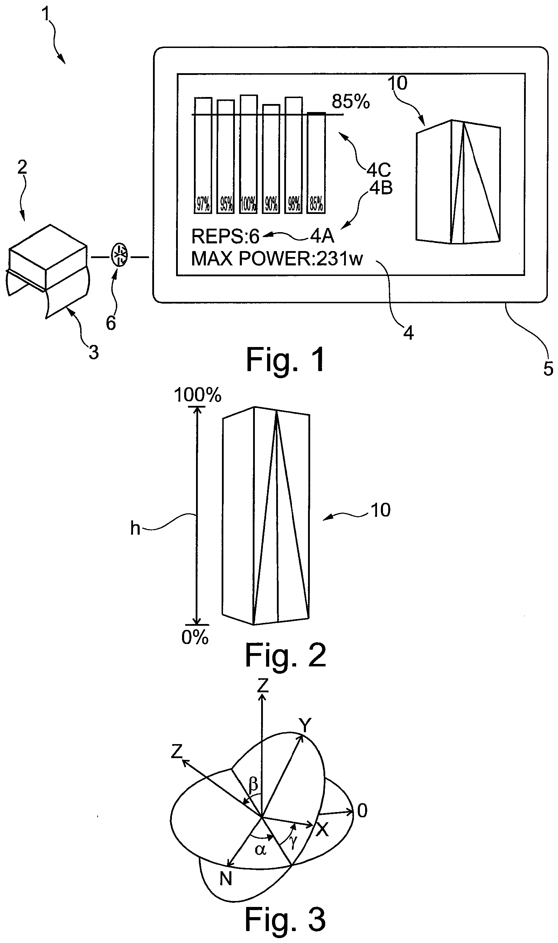

In the particular example of Figure 1, the display 4 and the processing means 5 are an integral part of an electronic device such as a tablet, a smartphone, a desktop computer, a notebook, a netbook, or the like.

Alternatively, the display 4 and the processing means 5 may be separate and distinct means. For example, the display 4 may be a TV screen and the processing means 5 consist of a personal computer, and they are in signal communication with each other.

The apparatus 1 has the purpose of optimizing the training session of an athlete according to his/her muscular characteristics.

To this end, the measuring device 2 comprises a transducer (or sensor) having at least one accelerometer. Particularly, the transducer preferably implements a MEMS (Micro Electro-Mechanical Systems) technology.

In a preferred embodiment of the present invention, in addition to the accelerometer the transducer also comprises a gyroscope and a magnetometer.

In this configuration, the measuring device 2 allows motion measurements along nine axes (see Figure 3). Namely, Figure 3 also represents the rotations in space considering the "world axes" coordinate system Z, O, N and the triplet of "fixed-body axes" x-y-z associated with the transducer, as well as the attitude angles $\alpha$, $\beta$, $\varphi$, also associated with the transducer.

Particularly, the apparatus 1 allows measurement of dynamic parameters (speed, acceleration, force, power, acceleration change rate) and, if a gyroscope and a magnetometer are also implemented in the transducer, also kinematic parameters (attitude), during the gymnastic exercise, with at least one value representative of the performance of the athlete appearing in real-time on the display 4.

For simplicity, the transducer of the measuring device 2 is assumed to implement the accelerometer, the gyroscope and the magnetometer, without limiting the general principle that simply requires acquisition of acceleration to carry out the steps of the inventive method.

Advantageously, the transducer is designed to generate a first sequence of values S1 representative of the movement of the body of the athlete or the equipment for performing the gymnastic exercise along any of the coordinate axes x, y, z.

Particularly, the first sequence of values S1 comprises first values S1' identifying the acceleration of the body of the athlete or the equipment for performing the gymnastic exercise along one of the axes x, y or z, and second values S1" identifying the attitude of the body of the athlete or the equipment for performing the gymnastic exercise.

When the gyroscope and the magnetometer are also implemented in the transducer, such transducer provides the first sequence of values S1 identifying, both acceleration along the three axes x, y, z, and the attitude of the body of the athlete or the instrument for performing the gymnastic exercise.

Particularly, the first sequence of values S1 provides the indication of acceleration along the three axes x, y, z (i.e. the fixed-body axes of the sensor) and the attitude (as Euler angles or quaternions).

The processing means 5 receive the first sequence of values S1 and are configured to determine, during the gymnastic exercise, the at least one value representative of the performance of the athlete as a function of the first sequence of values S1, to display such at least one value on the display 4.

For this purpose, the method of the present invention advantageously processes such first sequence of values S1 so received, and affords real-time display of training parameters.

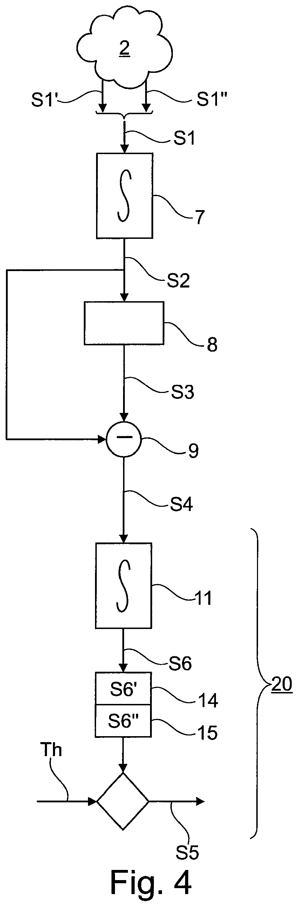

Figure 4 shows a flow diagram of a first embodiment of the method of the present invention.

Particularly, in order to carry out the inventive method, the apparatus 1 comprises an IT product and the processing means 5 comprise a memory (not shown), the IT product being adapted to be directly loaded into the memory of the processing means 5 and comprising program code portions which are adapted to carry out the inventive method when running on such processing means.

The starting point for such processing consists in determining, during the gymnastic exercise, the first sequence of values S 1, identifying the acceleration of the body of the athlete or the equipment for performing the gymnastic exercise.

It shall be noted that this acceleration measurement is affected by random oscillations caused by the operating equipment (here the combination accelerometer-gyroscope-magnetometer).

When trying to determine speed from this acceleration value through an integration process 7, to obtain a second sequence of values S2 identifying the speed of movement of the body of the athlete or the equipment for performing the gymnastic exercise, such second sequence of values S2 shows a drift error.

Particularly, even when the transducer is still, i.e. the body of the athlete or the equipment does not move, a non-zero speed value is still detected.

In an attempt to obviate this drift error, the second sequence of values S2 will be advantageously processed by calculating the least squares regression coefficients 8 to generate a third sequence of values S3 identifying the drift error trend of the measuring device 2.

A subtraction step 9 is also provided, in which such the third sequence of values S3 is subtracted from the second sequence of values S2, to obtain a fourth sequence of values S4, identifying the speed, without the drift error, at which the transducer moves during the exercise.

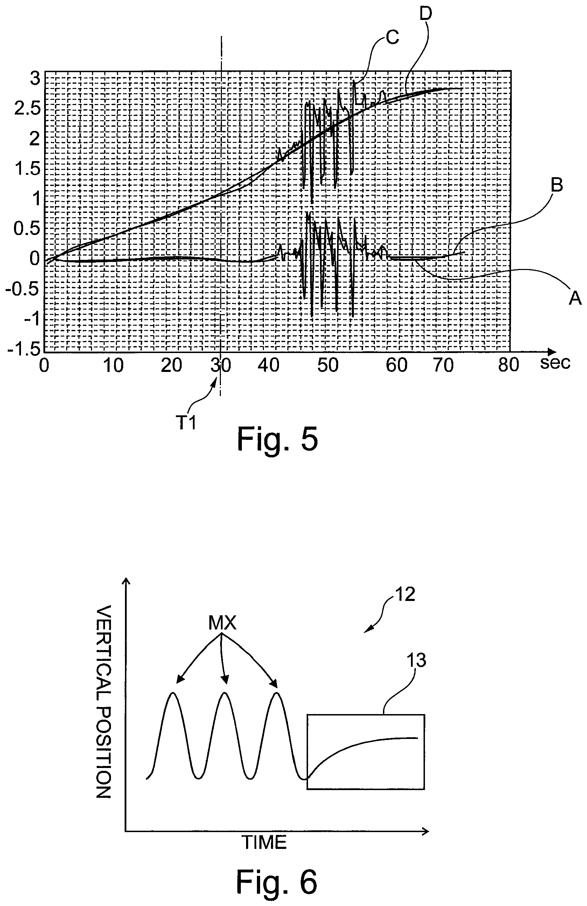

In other words, also referring to Figure 5, it may be noted that, until time T1, i.e. about the 30th second, the transducer is totally still (as shown by curve A) but, from an analysis of the second set of values S2, it appears to be moving (curve C).

In order to obviate this drawback, as mentioned above, the least squares regression coefficients 8 are calculated from the values S2, which will provide the third set of values S3 (curve D), which describes the drift error trend. Now, the drift trend curve (curve D) is subtracted (block 9) from the speed obtained by integration (curve C), thereby providing the correct speed (curve B), i.e. the sequence of values S4.

Once the sequence of values S4 has been obtained, a step is provided in which the athlete is informed, while he/she is performing the gymnastic exercise, about the at least one value representative of his/her performance, such at least one value being determined as a function of the fourth sequence of values S4.

Therefore, during the gymnastic exercise, the method of the invention extrapolates at least one value representative of the performance of the athlete, which is useful for immediate assessment of the exercise itself

For example, the value representative of the performance, as shown on the display 4, may be selected from the group of kinematic and dynamic parameters and bio-mechanical indicators.

Particularly, the value representative of the performance as shown on the display 4 may be the number of repetitions performed 4A, the maximum power 4B, the average power, the average force, the average speed and/or the maximum values attained.

This value representative of performance may be calculated from the fourth sequence of values S4, which represents the correct speed (i.e. without the drift error), at which the transducer moves during the exercise.

In order to inform the athlete, also referring to Figure 2, the apparatus may use a graphical icon 10, that is designed to appear on the display 4. This graphical icon 10 has, for example, a bar shape.

In a preferred embodiment, the height "h" of the bar 10 is adapted, for instance, to be proportional to the at least one value representative of performance. Thus, the athlete will simply watch the display 4 of the electronic device (PC, tablet, smartphone, or the like) to see in real time such at least one value representative of his/her performance.

The bar 10 is also used to inform the athlete in real time (i.e. as he/she performs the gymnastic exercise) about whether he/he has actually reached his/her training goal.

For example, still referring to the display 4 of Figure 1, if the athlete wants to increase his/her muscle mass (hypertrophy), the bar will display the power 4C, and at each thrust (also known as "repetition") on the equipment for performing the gymnastic exercise, to make his/her work effective, he/she will push at least to 90% his/her maximum power. Therefore, a threshold, here for instance 85%, will be combined to the bar 10 and graphically displayed, and a visual signal will be combined to the attainment or failed attainment of such threshold, such that during training the user will always know whether he/she has reached 85% his/her maximum power (and hence training is effective) or the threshold is not reached, which will indicate a bad an potentially ineffective training.

It shall be noted that the step of processing the second sequence of values S2 by calculating the least squares regression coefficients 8 to generate a third sequence of values S3, may be carried out on a limited number Nlim of values belonging to such second sequence of values S2 within a preset assessment range Tc.

In order to ensure real-time display of the at least one value representative of the performance of the athlete, such as force, speed, power, explositivy, etc. a limited number Nlim of values are selected, which means that this step of calculating the least squares regression coefficients 8 is applied within the preset assessment range Tc.

For example, a value of the preset assessment range Tc may be five seconds and, since a feasible acquisition frequency fc for the apparatus 1 is 50 Hz, this will mean that 250 values will be acquired, as fc=50Hz x Tc=5 sec = 250 values.

Therefore, in a preferred aspect, in order to avoid a computational overload on the processing means 5, a limited number Nlim of values are only selected within a preset assessment range Tc.

For example, this limited number Nlim of values within the assessment range Tc may be 50 values instead of 250, for calculation of the least squares regression coefficients 8.

This limited number Nlim of values are selected within the assessment range Tc, preferably in equally spaced fashion.

Turning back to the above disclosed numerical example, this will involve the acquisition of every tenth value of the buffer vector containing the speed values acquired in the last five seconds.

It shall be further noted that, in a preferred exemplary embodiment of the method, the step of subtracting the third sequence of values S3 from the second sequence of values S2 to obtain the fourth sequence of values S4 identifying the speed without the drift error, is not carried out on the entire sequence of values S3, i.e. for all the values acquired within the assessment range Tc, but at a single current time Tatt.

In other words, the subtraction step is carried out at a single time Tatt, identifying the current measuring time, the value of the single time of the second sequence of values S2 being subtracted from the respective value of said single time of said third sequence of values S3.

Therefore, the subtraction step may be schematically indicated as follows:

$$1)\quad S4\ (=\text{correct\_speed}\ (t))\ =\ S2\ (=\text{integrated\_speed}(t))\ -\ S3\ (=\text{regression\_curve}(t))$$

where "regression_curve" is a vector containing 250 data and relates to a time interval from Tatt to Tatt- Tc seconds and "integrated speed" is a vector that relates to a time interval from Tatt to Tatt- Tc seconds.

The result of the operation as designated above by (1), which is a subtraction of a single element of both vectors, is a scalar, i.e. the correct speed at time Tatt.

For further correction of the value, at the current time Tatt, further refinement is carried out by the following operation:

$$2)\quad S4(=\text{correct\_speed}\ (t\text{-}3))\ =\ S2(=\text{integrated\_speed}(t\text{-}3))\ -\ S3(=\text{regression\_speed}(t\text{-}3)).$$

After a first speed correction, under 1), a further correction is made after three times (under 2).

Thus, the introduction of a small delay will provide values that are even closer to the actual value. This is because correction is not made with reference to the last value of the buffer of the least squares regression curve "regression_curve(t)", but to a value "within" the buffer, regression_curve(t-3), which will be more "balanced".

From the speed data cleared of the drift effects ("drift") caused by the errors introduced by the measuring device 2, still referring to Figure 4, the repetitions performed by the athlete during the gymnastic exercise may be counted, and this value indicative of the performances of the athlete may be displayed on the display 4.

For this purpose, it shall be noted that the method comprises the additional step 20 of processing the fourth sequence of values S4 to obtain a value S5, identifying the number of repetitions performed by the athlete.

Particularly, integral calculation 11 is performed on the fourth sequence of values S4 to generate the value S5, identifying the number of repetitions.

It shall be noted that the sequences of values S1 to S4 are preferably related to a vertical axis, i.e. the axis of vertical movement of the body of the athlete or the equipment, as the axis z of the coordinate system x y, z.

If the sequence of values S4 is related to the axis z, i.e. the vertical axis, then the sequence of values S5 advantageously allows identification of a position measurement, namely height relative to a reference level (e.g. a reference plane or the starting position of the transducer), in which the transducer is located, always in real time.

Thus, also referring to Figure 5, it shall be noted that the measurement of the vertical position obtained, for instance, by three low-high-low-high-low-high-low movements of the transducer, includes a curve 12 drawn on a plane having time on the x-axis and the vertical position of the transducer on the y-axis. Particularly, the curve 12 is defined by the number of maxima "Mx" (in this special case the curve 12 has three maxima M, as three low-high-low-high-low-high low movements of the transducer have been made), which represent the number of repetitions performed by the athlete.

A fictitious "rise" is present at the end of the curve 12, as shown by the box 13. At these times of the box 13, the transducer is still in the position from which it started, then a horizontal line should have appeared in such box 13, instead of an "ascending" curve. This ascending curve in the box 13 may affect the computation of maxima Mx in the curve 12, and the number of repetitions displayed to the athlete will not match those actually performed.

In order to eliminate such "fictitious" rise of the transducer to define the actual number of repetitions performed by the athlete during the gymnastic exercise, the method of the present invention includes calculating the integral 11 of the sequence of values S4, to generate a sequence of values S6 representative of the movement made by the body of the athlete or the equipment for performing the gymnastic exercise.

Then, a portion of values S6' belonging to the sequence of values S6 is selected (block 14), and each value in such portion of values S6' is processed by calculating the standard deviation (block 14) to obtain a further sequence of processed values S6".

Each value of such sequence of values S6" is compared with a threshold value Th and if the values of such processed sequence S6" are higher than the threshold value Th, the value S5 representative of the number of repetitions performed by the athlete is generated.

Once the value S5 has been obtained, the athlete may be informed during his/her exercise about the number of repetitions performed to that moment, by means of the graphical icon 10.

It shall be noted that the threshold value Th, to be compared with each value of the series of values S6" is related to the weight of the equipment used by the athlete. Thus, any low-weight or bodyweight exercise movement (with low inertia), will be characterized by much higher standard deviation values on position measurement than those characterizing a high-weight movement, with high inertia.

Namely, the higher the threshold value Th the lower the weight value. For instance, the threshold value Th is supposedly 0.07 for a weight of more than 50 kg, or 0.12 for a weight of more than 15 kg, or 0.15 for a weight of more than 2 kg, or 0.2 for bodyweight exercise movements.

More in detail, in order to determine the number of repetitions S5, the method is designed to include a step in which the rise of the transducer (i.e. actually the increase of its height relative to a reference plane such as the ground) is recognized and the vertical position value, i.e. height, as measured at the first starting time of the rise is saved in a variable in the memory of the processing means 5. This value is stored in this memory and subtracted from the height value as measured at the end of the rising step. Thus, the space covered during the rise is estimated.

At the same time, during the rising step, the standard deviation of the vertical position is measured, and the maximum standard deviation that is reached as the weight rises is stored in a variable. Standard deviation is only calculated, for example, from the 18th acquisition of the rise (the data is acquired at a sampling rate fc of 50 Hz), to avoid influences by direction changes, where peaks are always found, as standard deviation is calculated, for example, from the last twenty-five values.

Once a rising step and the maximum standard deviation value associated therewith have been determined, a repetition is found to have been performed if displacement is greater, for instance, than 0.15 meters and standard deviation remains above a given threshold Th or is zero (which is the case of a very fast rise, lasting less than 18 acquisitions, i.e. 18 acquisitions / 50 Hz = 0.36 seconds).

If the measured rise is greater than 0.15 meters and the standard deviation is below a given threshold Th, the repetition is not counted, because it is not an actual movement, but the undesired "fictitious" rise effect as shown in the above box 13.

Once the fourth sequence of values S4 has been found, the attitude of the transducer and the body of the athlete and the equipment he/she uses may be controlled by the gyroscope, to acquire information about the attitude in space. For this purpose, the fourth sequence S4 is processed to compare the attitude angles at the start of and throughout the exercise. If these values change by a give threshold, then the athlete is informed. This may be useful to monitor any movement irregularity (e.g. an athlete that might excessively move a barbell as he/she performs a give exercise, which may be monitored).

Once the value S5 has been obtained, the regularity of repetitions may be monitored. The analysis of maximum power values within an entire series (by processing the fourth sequence of values S4), allows determination of variations and analysis of the quality of the repetitions performed.

As a rule, the athlete tends to always perform the same movement, until his/her muscular capacity wears out as the series goes on. Nevertheless, the muscular capacity of the athlete may happen to always exceed the preset threshold without deteriorating with time. In this case, the method may advise the user to increase the weight. On the other hand, when very different maximum power measurements are found for the athlete during a series, the method may inform the athlete that he/she is making a wrong movement or is using an excessively high weight.

Once the fourth sequence of values S4 is obtained, the stability of the core and the performance of the exercise may be analyzed. Particularly, the core of the athlete is the anatomic region that corresponds to the torso and transfers forces from the lower limbs to the upper limbs of the skeleton (or vice versa). It is often a limiting factor for athletic performance and one of the most important parameters for injury prevention. An athlete under a barbell will find it more difficult to stabilize weight as the muscles of his/her core are weaker.

A core strength estimation may be obtained based on the stability with which an athlete moves when he/she is handling weights during an exercise. The method allows direct stability measurement by determining the standard deviation of accelerations on the plane XY (i.e. the plan corresponding to the floor), in the vertical direction, and attitude variations during the exercise. In this case, the sensor will have to be placed on the core of the athlete and not on the weight.

Furthermore, stability may be also checked for quasi-isometric exercises (i.e. particular types of exercises performed while maintaining positions with special joint angles for a given time), as the effectiveness of the isometric exercise is assessed exactly like core strength, i.e. using the standard deviation of accelerations in space and monitoring attitude variations with respect to the initial attitude, using the angles measured by the gyroscope on the sensor. The monitoring parameters are shown on the display.

Referring now to Figure 8, a further flow diagram is shown, in which parts or steps that have been already described are designated by the same numerals. This flow diagram schematically shows an alternative embodiment of the present invention. Particularly, in this alternative embodiment the least squares regression coefficients 8 are calculated on the first sequence of values S1 to generate a processed sequence S7 and a further subtraction step is performed to subtract the sequence of values S7 from the first sequence of values A1 to generate a new sequence of values S8 cleared of the drift error. This sequence S8 is integrated (block 7) to determine the sequence of values S4 identifying the at least one value representative of performance.

Those skilled in the art will obviously appreciate that a number of changes and variants may be made to the embodiments of the method and apparatus for assessing the performances of an athlete that performs a gymnastic exercise as described hereinbefore to meet specific needs, without departure from the scope of the invention, as defined in the following claims.

**Claims**

**1.** A method of assessing the performances of an athlete that performs a gymnastic exercise, comprising the steps of:

- providing a measuring device and removably associating said measuring device to the body of the athlete or to equipment for performing said gymnastic exercise, said measuring device comprising a transducer having at least one accelerometer; and, using processing means:

a) determining, during the gymnastic exercise, a first sequence of values (S1), identifying the acceleration of said body of the athlete or said equipment for performing said gymnastic exercise, along a preset axis (x,y,z);
b) processing said first sequence of values (S1) by integral calculation (7) to obtain a second sequence of values (S2) identifying speed;
c) processing said second sequence of values (S2) by calculating the least squares regression coefficients (8) to generate a third sequence of values (S3);
d) subtracting said third sequence of values (S3) from said second sequence of values (S2) to obtain a fourth sequence of values (S4) identifying the speed at which said body of the athlete or said equipment for performing said gymnastic exercise moves;

e) informing said athlete, during said gymnastic exercise, about at least one value representative of said performance of said athlete, said at least one value being determined as a function of said fourth sequence of values

f) processing said fourth sequence of values (S4) by integral calculation (7) to obtain a repetition value (S5) identifying the number of repetitions performed during said gymnastic exercise;

**characterized in that** said step f) comprises the steps of:

f1) providing a threshold value (Th);

f2) processing said fourth sequence of values (S4) by integral calculation (7) to obtain a sixth sequence of values (S6);

f3) selecting a portion of values (S6') belonging to said sixth sequence of values (S6);

f4) processing said portion of the sequence of values (S6') by standard deviation calculation (15) to obtain a sequence of processed values (S6");

f5) comparing said threshold value (Th) with each value of said sequence of processed values (S6") and, if said processed value is higher than said threshold value (Th), generating said repetition value (S5);

f6) informing said athlete, during said gymnastic exercise, about said repetition value representative of the number of repetitions performed by said athlete during said gymnastic exercise.

2. A method of assessing the performances of an athlete that performs a gymnastic exercise as claimed in claim 1, wherein said step a) comprises the steps of:

a1) receiving first values (S1') identifying the acceleration of said body of the athlete or said equipment for performing said gymnastic exercise, from said measuring device during said gymnastic exercise, said first values (S1') being detected as a function of the axes of said measuring device;

a2) receiving second values (S1") identifying the attitude of said body of the athlete or said equipment for performing said gymnastic exercise, from said measuring device during said gymnastic exercise;

a3) processing said first values (S1') with said second values (S1") to generate said first sequence of values (S1).

3. A method of assessing the performances of an athlete that performs a gymnastic exercise as claimed in claim 1 or 2, wherein said step c) is carried out on a limited number (Nlim) of values belonging to said second sequence of values (S2) within a preset assessment range (Tc), said range having a shorter duration than the overall duration of said gymnastic exercise.

4. A method of assessing the performances of an athlete that performs a gymnastic exercise as claimed in claim 1, wherein said step d) is carried out at a single time (Tatt), identifying the current measuring time, the value of said single time of said second sequence of values (S2) being subtracted from the respective value of said single time of said third sequence of values (S3).

5. A method of assessing the performances of an athlete that performs a gymnastic exercise as claimed in any of the preceding claims, wherein said accelerometer is a three-axis accelerometer, and said transducer comprises a gyroscope and a magnetometer.

6. A method of assessing the performances of an athlete that performs a gymnastic exercise as claimed in any of the preceding claims, wherein said at least one value representative of said performances of said athlete is selected from the group comprising kinematic and dynamic parameters and bio-mechanical indicators.

7. An apparatus for assessing the performances of an athlete that performs a gymnastic exercise, comprising:

- a measuring device (2);

- fastener means (3) for removably fastening said measuring device (2) to the body of the athlete or to equipment for performing said gymnastic exercise;

- said measuring device (2) comprising a transducer having at least one accelerometer, the latter being designed to generate a first sequence of values (S1) representative of acceleration along an axis of movement of the body of the athlete or the equipment for performing said gymnastic exercise;

- a display (4);

- processing means (5) in signal communication with said measuring device (2) and with said display (4) said processing means (5) receiving said first sequence of values (S1) and being designed to determine, during said gymnastic exercise, at least one value representative of said performance of said athlete as a function of said first sequence of values, to display said at least one value on said display;

**characterized in that** sad apparatus (1) comprises an IT product and said processing means (5) comprise a memory, said IT product being adapted to be directly loaded into the memory of processing means (5) and comprising program code portions which are adapted to carry out the method as claimed in any of claims 1 to 6 when running on said processing means.

8. An apparatus for assessing the performances of an athlete that performs a gymnastic exercise as claimed in claim 7, wherein said measuring device comprises a radio-frequency transceiver module (6).

9. An apparatus for assessing the performances of an athlete that performs a gymnastic exercise as claimed in claim 8, wherein said radio-frequency transceiver module (6) is a wireless communication module using the Bluetooth protocol.

10. An apparatus for assessing the performances of an athlete that performs a gymnastic exercise as claimed in claim 7, wherein said fastener means (3) comprise a magnetic coupling.

11. An apparatus for assessing the performances of an athlete that performs a gymnastic exercise as claimed in claim 7, wherein said measuring device (2) is a self-powered device.

**Patentansprüche**

1. Verfahren zur Bewertung der Leistungen eines Sportlers, der eine Gymnastikübung ausführt, umfassend folgende Schritte:

- Bereitstellen einer Messvorrichtung und lösbares Verbinden der Messvorrichtung mit dem Körper des Sportlers oder mit der Ausrüstung zur Ausführung der Gymnastikübung, wobei die Messvorrichtung einen Aufnehmer mit wenigstens einem Beschleunigungsmesser umfasst;
und, unter Einsatz von Verarbeitungsmitteln:

a) Erfassen - während der Gymnastikübung - einer ersten Wertsequenz (S1) zur Bestimmung der Beschleunigung des Körpers des Sportlers oder der Ausrüstung zur Ausführung der Gymnastikübung längs einer voreingestellten Achse (x,y,z);
b) Verarbeiten der ersten Wertsequenz (S1) durch integrale Berechnung (7), um eine zweite Wertsequenz (S2) zur Bestimmung der Geschwindigkeit zu erhalten;
c) Verarbeiten der zweiten Wertsequenz (S2) durch Berechnung der Regressionskoeffizienten der kleinsten Quadrate (8), um eine dritte Wertsequenz (S3) zu erzeugen;
d) Subtrahieren der dritten Wertsequenz (S3) von der zweiten Wertsequenz (S2), um eine vierte Wertsequenz (S4) zur Bestimmung der Geschwindigkeit zu erhalten, mit der Körper des Sportlers oder die Ausrüstung zur Ausführung der Gymnastikübung sich bewegt;
e) Informieren des Sportlers während der Gymnastikübung über wenigstens einen Wert, der für die Leistung des Sportlers repräsentativ ist, wobei der wenigstens eine Wert in Abhängigkeit der vierten Wertsequenz festgelegt wird;
f) Verarbeiten der vierten Wertsequenz (S4) durch integrale Berechnung (7), um einen Wiederholungswert (S5) zur Bestimmung der Anzahl von Wiederholungen zu erhalten, die während der Gymnastikübung ausgeführt werden;

**dadurch gekennzeichnet, dass** der Schritt f) folgende Schritte umfasst:

f1) Bereitstellen eines Schwellwerts (Th);
f2) Verarbeiten der vierten Wertsequenz (S4) durch integrale Berechnung (7),
um eine sechste Wertsequenz (S6) zu erhalten;
f3) Auswählen eines Bereichs von Werten (S6'), die zur sechsten Wertsequenz (S6) gehören;
f4) Verarbeiten des Bereichs der Wertsequenz (S6') durch Berechnung der Standardabweichung (15), um eine Sequenz verarbeiteter Werte (S6") zu erhalten;

f5) Vergleichen der Schwellwerte (Th) mit jedem Wert der Sequenz verarbeiteter Werte (S6") und, wenn der verarbeitete Wert höher als der Schwellwert (Th) ist, Erzeugen des Wiederholungswerts (S5);

f6) Informieren des Sportlers während der Gymnastikübung über den Wiederholungswert, der repräsentativ für die Anzahl der vom Sportler während der Gymnastikübung vorgenommenen Wiederholungen ist.

2. Verfahren zur Bewertung der Leistungen eines Sportlers, der eine Gymnastikübung ausführt, nach Anspruch 1, wobei der Schritt a) folgende Schritte umfasst:

a1) Empfangen erster Werte (S1') zur Bestimmung der Beschleunigung des Körpers des Sportlers oder der Ausrüstung zur Ausführung der Gymnastikübung von der Messvorrichtung während der Gymnastikübung, wobei die ersten Werte (S1') in Abhängigkeit der Achsen der Messvorrichtung erfasst werden;

a2) Empfangen zweiter Werte (S1") zur Bestimmung der Haltung des Körpers des Sportlers oder der Ausrüstung zur Ausführung der Gymnastikübung von der Messvorrichtung während der Gymnastikübung;

a3) Verarbeiten der ersten Werte (S 1') mit den zweiten Werten (S1"), um die erste Wertsequenz (S1) zu erzeugen.

3. Verfahren zur Bewertung der Leistungen eines Sportlers, der eine Gymnastikübung ausführt, nach Anspruch 1 oder 2, wobei der Schritt c) mit einer begrenzten Anzahl (Nlim) von Werten, die zu der zweiten Wertsequenz (S2) gehören, innerhalb eines voreingestellten Bewertungsbereichs (Tc) ausgeführt wird, wobei der Bereich eine kürzere Dauer als die Gesamtdauer der Gymnastikübung aufweist.

4. Verfahren zur Bewertung der Leistungen eines Sportlers, der eine Gymnastikübung ausführt, nach Anspruch 1, wobei der Schritt d) zu einem einzelnen, die laufende Messzeit bestimmenden Zeitpunkt (Tatt) ausgeführt wird, wobei der Wert des einzelnen Zeitpunkts der zweiten Wertsequenz (S2) von dem jeweiligen Wert des einzelnen Zeitpunkts der dritten Wertsequenz (S3) abzuziehen ist.

5. Verfahren zur Bewertung der Leistungen eines Sportlers, der eine Gymnastikübung ausführt, nach einem der vorstehenden Ansprüche, wobei der Beschleunigungsmesser ein Dreiachsen-Beschleunigungsmesser ist und der Aufnehmer einen Gyroskopen und ein Magnetometer umfasst.

6. Verfahren zur Bewertung der Leistungen eines Sportlers, der eine Gymnastikübung ausführt, nach einem der vorstehenden Ansprüche, wobei wenigstens ein Wert, der repräsentativ für die Leistungen des Sportlers ist, ausgewählt ist aus der Gruppe umfassend kinematische und dynamische Parameter und biomechanische Kennzahlen.

7. Vorrichtung zur Bewertung der Leistungen eines Sportlers, der eine Gymnastikübung ausführt, umfassend:

- eine Messvorrichtung (2);
- Befestigungsmittel (3) zum lösbaren Befestigen der Messvorrichtung (2) an dem Körper des Sportlers oder an der Ausrüstung zur Ausführung der Gymnastikübung;
- wobei die Messvorrichtung (2) einen Aufnehmer mit wenigstens einem Beschleunigungsmesser umfasst, wobei Letzterer dazu bestimmt ist, eine erste Wertsequenz (S1) zu erzeugen, die repräsentativ für die Beschleunigung längs einer Bewegungsachse des Körpers des Sportlers oder der Ausrüstung zur Ausführung der Gymnastikübung ist;
- eine Anzeige (4);
- Verarbeitungsmittel (5) in Signalverbindung mit der Messvorrichtung (2) und mit der Anzeige (4), wobei die Verarbeitungsmittel (5) eine erste Wertsequenz (S1) empfangen und dazu bestimmt sind, während der Gymnastikübung wenigstens einen Wert zu ermitteln, der repräsentativ für die Leistung des Sportlers in Abhängigkeit der ersten Wertsequenz ist, um den wenigstens einen Wert auf der Anzeige anzuzeigen;

**dadurch gekennzeichnet, dass** die Vorrichtung (1) ein IT-Produkt umfasst und die Verarbeitungsmittel (5) einen Speicher umfassen, wobei das IT-Produkt dazu geeignet ist, direkt in den Speicher der Verarbeitungsmittel (5) geladen zu werden und Programmcodeteile umfasst, die dazu geeignet sind, das nach einem der Ansprüche 1 bis 6 beanspruchte Verfahren beim Ablaufen auf den Verarbeitungsmitteln auszuführen.

8. Vorrichtung zur Bewertung der Leistungen eines Sportlers, der eine Gymnastikübung ausführt, nach Anspruch 7, wobei die Messvorrichtung ein Funkfrequenzempfängermodul (6) umfasst.

9. Vorrichtung zur Bewertung der Leistungen eines Sportlers, der eine Gymnastikübung ausführt, nach Anspruch 8, wobei das Funkfrequenzempfängermodul (6) ein drahtloses Kommunikationsmodul ist, welches das Bluetooth-Protokoll verwendet.

10. Vorrichtung zur Bewertung der Leistungen eines Sportlers, der eine Gymnastikübung ausführt, nach Anspruch 7, wobei die Befestigungsmittel (3) eine magnetische Kupplung umfassen.

11. Vorrichtung zur Bewertung der Leistungen eines Sportlers, der eine Gymnastikübung ausführt, nach Anspruch 7, wobei die Messvorrichtung (2) eine selbstversorgende Vorrichtung ist.

**Revendications**

1. Un procédé d'évaluation des performances d'un athlète qui effectue un exercice de gymnastique, comprenant les étapes de :

   - fournir un dispositif de mesure et associer de façon détachable ledit dispositif de mesure au corps de l'athlète ou à l'équipement pour effectuer ledit exercice de gymnastique, ledit dispositif de mesure comprenant un transducteur ayant au moins un accéléromètre;

   et, à l'aide d'un moyen de traitement :

   a) déterminer, durant l'exercice de gymnastique, une première séquence de valeurs (S1), identifiant l'accélération dudit corps de l'athlète ou dudit équipement permettant d'effectuer ledit exercice de gymnastique, le long d'un axe prédéfini (x,y,z);
   b) traiter ladite première séquence de valeurs (S1) par calcul d'intégrale (7) pour obtenir une deuxième séquence de valeurs (S2) identifiant la vitesse;
   c) traiter ladite deuxième séquence de valeurs (S2) par le calcul des coefficients de régression des moindres carrés (8) afin de générer une troisième séquence de valeurs (S3);
   d) soustraire ladite troisième séquence de valeurs (S3) de ladite deuxième séquence de valeurs (S2) pour obtenir une quatrième séquence de valeurs (S4) identifiant la vitesse à laquelle ledit corps de l'athlète ou ledit équipement permettant d'effectuer l'exercice de gymnastique se déplace;
   e) informer ledit athlète, durant ledit exercice de gymnastique, d'au moins une valeur représentative de ladite performance dudit athlète, ladite au moins une valeur étant déterminée comme une fonction de ladite quatrième séquence de valeurs
   f) traiter la quatrième séquence de valeurs (S4) par calcul d'intégral (7) pour obtenir une valeur de répétition (S5) identifiant le nombre de répétitions effectuées durant ledit exercice de gymnastique;
   **caractérisé en ce que** ladite étape f) comprend les étapes consistant à :

   f1) fournir une valeur de seuil (Th);
   f2) traiter ladite quatrième séquence de valeurs (S4) par calcul d'intégrale (7) pour obtenir une sixième séquence de valeurs (S6);
   f3) sélectionner une portion de valeurs (S6') appartenant à ladite sixième séquence de valeurs (S6);
   f4) traiter ladite portion de la séquence de valeurs (S6') par calcul d'écart type (15) pour obtenir une séquence de valeurs traitées (S6");
   f5) comparer ladite valeur de seuil (Th) avec chaque valeur de ladite séquence de valeurs traitées (S6") et, si ladite valeur traitée est supérieure à ladite valeur de seuil (Th), générer ladite valeur de répétition (S5);
   f6) informer ledit athlète, durant ledit exercice de gymnastique, sur ladite valeur de répétition représentative du nombre de répétitions effectuées par ledit athlète durant ledit exercice de gymnastique.

2. Procédé d'évaluation des performances d'un athlète qui effectue un exercice de gymnastique, tel que défini dans la revendication 1, ladite étape a) comprend les étapes consistant à:

   a1) recevoir de premières valeurs (S1') identifiant l'accélération dudit corps de l'athlète ou dudit équipement permettant d'effectuer ledit exercice de gymnastique, à partir dudit dispositif de mesure durant ledit exercice de gymnastique, lesdites premières valeurs (S1') étant détectées comme une fonction des axes dudit dispositif de mesure;
   a2) recevoir de deuxièmes valeurs (S1") identifiant l'attitude dudit corps de l'athlète ou dudit équipement permettant d'effectuer ledit exercice de gymnastique, à partir dudit dispositif de mesure durant ledit exercice de gymnastique;

a3) traiter lesdites premières valeurs (S1') avec lesdites deuxièmes valeurs (S1") pour générer ladite première séquence de valeurs (S1).

3. Procédé d'évaluation des performances d'un athlète qui effectue un exercice de gymnastique tel que défini dans la revendication 1 ou 2, ladite étape c) étant effectuée sur un nombre limité (Nlim) de valeurs appartenant à ladite deuxième séquence de valeurs (S2) dans un intervalle d'évaluation prédéfini (Tc), ledit intervalle ayant une durée plus courte que la durée totale dudit exercice de gymnastique.

4. Procédé d'évaluation des performances d'un athlète qui effectue un exercice de gymnastique tel que défini dans la revendication 1, ladite étape d) étant effectuée une seule fois (Tatt), identifiant la durée de mesure courante, la valeur de ladite seule fois de ladite deuxième séquence de valeurs (S2) étant soustraite de la valeur respective de ladite seule fois de ladite troisième séquence de valeurs (S3).

5. Procédé d'évaluation des performances d'un athlète qui effectue un exercice de gymnastique tel que défini dans l'une quelconque des revendications précédentes, dans lequel ledit accéléromètre est un accéléromètre à trois axes, et ledit transducteur comprend un gyroscope et un magnétomètre.

6. Procédé d'évaluation des performances d'un athlète qui effectue un exercice de gymnastique tel que défini dans l'une quelconque des revendications précédentes, dans lequel ladite au moins une valeur représentative desdites performances dudit athlète est sélectionnée à partir du groupe comprenant des paramètres cinématiques et dynamiques et des indicateurs biomécaniques.

7. Un appareil d'évaluation des performances d'un athlète qui effectue un exercice de gymnastique, comprenant :

   - un dispositif de mesure (2);
   - un moyen de fixation (3) permettant de fixer de façon détachable ledit dispositif de mesure (2) au corps de l'athlète ou à l'équipement permettant d'effectuer ledit exercice de gymnastique;
   - ledit dispositif de mesure (2) comprenant un transducteur ayant au moins un accéléromètre, ce dernier étant conçu pour générer une première séquence de valeurs (S1) représentative de l'accélération sur un axe de mouvement du corps de l'athlète ou l'équipement permettant d'effectuer ledit exercice de gymnastique;
   - un écran d'affichage (4);
   - un moyen de traitement (5) communiquant par signaux avec ledit dispositif de mesure (2) et avec ledit écran d'affichage (4), ledit moyen de traitement (5) recevant ladite première séquence de valeurs (S1) et étant conçu pour déterminer, durant ledit exercice de gymnastique, au moins une valeur représentative desdites performances dudit athlète comme une fonction de ladite première séquence de valeurs, pour afficher ladite au moins une valeur sur ledit écran d'affichage;

   **caractérisé en ce que** ledit appareil (1) comprend un produit TI et ledit moyen de traitement (5) comprend une mémoire, ledit produit TI étant adapté à être directement chargé dans la mémoire du moyen de traitement (5) et comprenant des portions de code de programme qui sont adaptés à réaliser le procédé tel que défini dans l'une quelconque des revendications de 1 à 6 lors de son exécution sur ledit moyen de traitement.

8. Appareil d'évaluation des performances d'un athlète qui effectue un exercice de gymnastique, tel que défini dans la revendication 7, dans lequel ledit dispositif de mesure comprend un module d'émetteur-récepteur radiofréquence (6).

9. Appareil d'évaluation des performances d'un athlète qui effectue un exercice de gymnastique, tel que défini dans la revendication 8, dans lequel ledit module d'émetteur-récepteur radiofréquence (6) est un module de communication sans fil utilisant le protocole Bluetooth.

10. Appareil d'évaluation des performances d'un athlète qui effectue un exercice de gymnastique, tel que défini dans la revendication 7, dans lequel ledit moyen de fixation (3) comprend un couplage magnétique.

11. Appareil d'évaluation des performances d'un athlète qui effectue un exercice de gymnastique, tel que défini dans la revendication 7, dans lequel ledit dispositif de mesure (2) est un dispositif autoalimenté.

Fig. 1

Fig. 2

Fig. 3

Fig. 4

Fig. 5

Fig. 6

Fig. 7

**EP 3 057 505 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1834583 A

**Non-patent literature cited in the description**

- **K. TAKENOSHITA et al.** Development of a Portable Acceleration Monitor Device and its clinical application for the Quantitative Gait Assessment of the Elderly. *Proceedings of the 2005 IEEE Engineering in Medicine and Biology 27th Annual Conference,* 3534-3537